# EUROPEAN PATENT SPECIFICATION

(11) **EP 2 056 717 B1**
(45) Date of publication and mention of the grant of the patent: **04.07.2012**
(21) Application number: 07801757.1
(22) Date of filing: 18.08.2007
(51) Int. Cl.: A61B 5/151

(54) **ELASTOMERIC TOROIDAL RING FOR BLOOD EXPRESSION**
ELASTOMERER TOROIDALER RING ZUM AUSDRÜCKEN VON BLUT
ANNEAU TOROIDAL EN ELASTOMERE POUR LE PRELEVEMENT DE SANG

(30) Priority: 22.08.2006 US 466202
(43) Date of publication of application: 13.05.2009
(73) Proprietor: Roche Diagnostics GmbH, 68305 Mannheim (DE); F. Hoffmann-La Roche AG, 4070 Basel (CH)
(72) Inventor: PATEL, Paul, Sunnyvale, CA 94086 (US); WONG, Daniel, Sunnyvale, CA 94087 (US)
(86) International application number: PCT/EP2007/007317
(87) International publication number: WO 2008/022755

(56) References cited:
- WO-A-01/89383
- WO-A-2004/045375
- WO-A-2005/112763
- US-B1- 6 306 104

## Description

### BACKGROUND

The present invention generally relates to an expression device and more specifically, but not exclusively, concerns an elastomeric toroidal ring for expressing body fluid from an incision.

The acquisition and testing of bodily fluids is useful for many purposes and continues to grow in importance for use in medical diagnosis and treatment, such as for diabetes, and in other diverse applications. In the medical field, it is desirable for lay operators to perform tests routinely, quickly, and reproducibly outside of a laboratory setting, with rapid results and a readout of the resulting test information. Testing can be performed on various bodily fluids and, for certain applications, is particularly related to the testing of blood and/or interstitial fluid.

Performing home diagnostics can sometimes be a painful experience. While certain locations on the body provide ample blood supplies, such as the fingers, these locations tend to have high nerve densities, which in turn can sometimes make lancing to collect the sample a very painful experience. Alternate body sites, such as the forearm and legs, have lower nerve densities, and thus the patient experiences less pain when lancing those sites. However, alternate sites also typically supply less blood than the fingers. Having deeper penetration depths when lancing alternate sites can sometimes increase the blood flow from the incision. However, lancing at too great of depths tends to be more painful. Therefore, it is desirable to minimize the pain associated with lancing the skin for home diagnostic testing. The minimal sample volumes needed for accurate testing have reduced over the years to the point where less than 1 µL samples volumes are common. However, most alternate sites still do not reliably produce sufficient quantities of blood to ensure consistently accurate results with minimal pain. Similarly, shallow incisions on the fingers, which tend to minimize pain, still do not reliably bleed sufficient volumes of blood for consistent test results.

Various design solutions have been proposed to increase or enhance the bleeding from incisions. In one technique, the incision site is pressed against an expression member, or vice versa, in order to locally increase the blood pressure inside the capillary channels that feed blood to the incision, thereby increasing bleeding from the incision. The results from this pressing technique are sometimes inconsistent, such that an insufficient sample for testing purposes is bled from the incision. When the fluid sample is insufficient, the patient then has to re-lance another site and continue the process again, which can be quite painful. Therefore, it is desirable to have an expression device that is able to consistently provide a sufficient fluid sample for testing purposes.

Thus, needs remain for further contributions in this area of technology.

### SUMMARY

One aspect concerns an expression device. The expression device includes an expression cap that defines a lancet opening. The expression cap has a skin contacting surface that is generally flat and positioned to face tissue during expression of body fluid. The expression cap includes an elastomeric ring extending around the lancet opening from the skin contacting surface. The elastomeric ring includes elastomeric material. The expression cap has a beveled surface between the lancet opening and the elastomeric ring to enhance expression of the body fluid when the expression cap is pressed around an incision site. The expression cap further has an overmold layer covering the elastomeric ring and inside the elastomeric ring the expression cap has a beveled surface.

Furthermore a method for expressing fluid is described. With the method, an incision is formed in skin. Body fluid is expressed from the incision by pressing around the incision in the skin an expression cap that includes an elastomeric ring made from elastomeric material and a beveled surface located inside the elastomeric ring.

Further forms, objects, features, aspects, benefits, advantages, and embodiments of the present invention will become apparent from a detailed description and drawings provided herewith.

### BRIEF DESCRIPTION OF THE DRAWINGS

FIG. 1 is a top plan view of an expression device.
FIG. 2 is a side perspective view of the FIG. 1 expression device.
FIG. 3 is a second top plan view of the FIG. 1 expression device that identifies the various dimensions in the device.
FIG. 4 is a side view of a variation of the FIG. 1 expression device that includes an overmold layer.
FIG. 5 is a cross-sectional view of the FIG. 1 expression device.

### DESCRIPTION OF THE SELECTED EMBODIMENTS

For the purposes of promoting an understanding of the principles of the invention, reference will now be made to the embodiments illustrated in the drawings and specific language will be used to describe the same. It will nevertheless be understood that no limitation of the scope of the invention is thereby intended, such alterations and further modifications in the illustrated device, and such further applications of the principles of the invention as illustrated therein being contemplated as would normally occur to one skilled in the art to which the invention relates. A number of embodiments of the invention are shown in detail; although it will be apparent to those skilled in the relevant art that some features that are not relevant to the present invention may not be shown for the sake of clarity. It should be noted that directional terms, such as "up", "down", "top" and "bottom", are used herein solely for the convenience of the reader in order to aid in the reader's understanding of the illustrated embodiments, and it is not the intent that the use of these directional terms in any manner limit the described, illustrated, and/or claimed features to a specific direction or orientation.

The present invention generally concerns an expression device for expressing fluid from an incision. The expression device includes an elastomeric ring, such as an o-ring, that is mounted to an expression plate. In one form, the expression device can have a unitary structure, and in another form, the device can have a two-component, complementary structure, such as an o-ring mounted to a support body. For the single molded piece, the expression device can be fabricated from a single material, such as silicone or other types of flexible elastomeric plastics, or a hard plastic, like polystyrene or ABS. For the two-component form, the expression device can be fabricated from two or more materials. For instance, the o-ring can be fabricated from an elastomeric material, and the support can be fabricated from a hard plastic with an elastomeric overmold. In another embodiment the support can be made from an elastomeric plastic. In one particular embodiment, the o-ring has an internal diameter of 7 mm and a cross-sectional width of 1.5 mm. The support in this embodiment defines a lancet opening that has an internal diameter of 6 mm. The support has a countersink or beveled surface around the lancet opening. The countersink can have an angle of 20° to 30°. For an expression device having a concave expression ring with a countersink angle of 20°, it was discovered that the expression device unexpectedly improved expression of blood by 148.1% as compared to a control expression device.

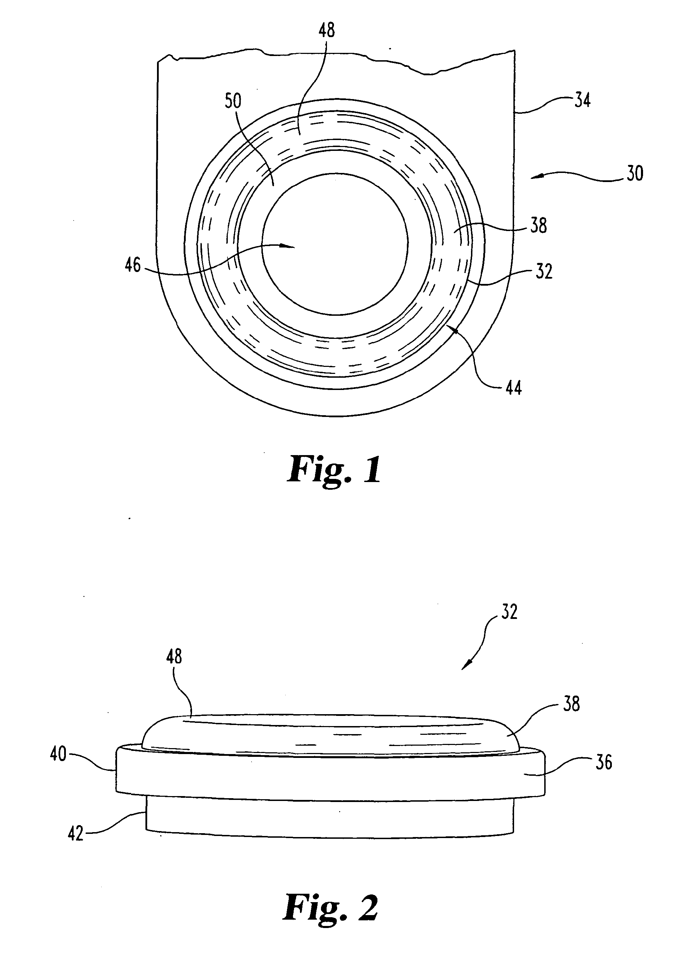

FIG. 1 illustrates an expression device 30 according to one embodiment, among many, of the present invention. As can be seen, the expression device 30 includes an expression cap 32 that is coupled to an expression body or plate 34. It should be appreciated that the expression cap 32 can be installed on other devices. For example, the expression cap 32 can be installed on a lancing device or on an integrated device, to name a few. It is also contemplated that various features of the expression device can be adapted for other systems. Nonlimiting examples of some systems in which the expression device 30 can be adapted for use are illustrated and described in the following: U.S. Patent No. 5,879,311 to Duchon et al., issued March 9, 1999; U.S. Published Application No. 2004/0127818 A1 to Roe et al., published July 1, 2004; U.S. Published Application No. 2003/0050573 A1 to Kuhr et al., published March 13, 2003; and U.S. Published Application No. 2003/0018282 A1 to Effenhauser et al., published January 23, 2003.

Turning to FIG. 2, the expression cap 32 includes a base 36 and an expression ring 38. The base 36 has a flange 40 upon which the expression ring 38 rests. The flange 40 extends from a collar 42. The collar 42 is configured to be received in a plate opening 44 in the expression plate 34 (FIG. 1). It should be appreciated, however, that the collar 42 can be received into other devices, such as lancing devices. Referring again to FIG. 1, the expression cap 32 is generally ring shaped and defines an expression or lancet opening 46 through which a piercing device, such as a lancet or needle, extends during lancing and where a fluid sample is collected. Although the expression cap and its various components are ring shaped, it is contemplated that the expression cap and its components can have a different overall shape. As mentioned previously, the expression device 30 in one form can have a unitary structure in which the expression cap 32 and the expression plate 34 are formed as a single structure. In other embodiments, the expression device 30 can have two or more components in a complementary structure. In the illustrated embodiment, the expression ring 38 is in the form of an o-ring attached to the expression flange 40. For a single molded unitary embodiment, the expression device 30 can be fabricated from a single material, such as silicone or other elastomeric plastics, or a hard plastic, such as polystyrene or ABS. When in a multicomponent form, the expression device 30 can be fabricated from one or more materials. For example, in one embodiment, the o-ring can be fabricated from elastomeric material and the expression cap 32 can be fabricated from a hard plastic. It should be recognized that other types of material combinations can be used in other embodiments.

Referring again to FIG. 1, the expression ring 38 has an outer contact surface 48 that is generally curved and configured to contact the skin during expression. Inside the expression ring 38, the expression cap 32 has a beveled or countersink surface 50 that surrounds the expression opening 46. As will be explained below, it was discovered that a 20° angle for the beveled surface 50 provided unexpected, exceptional results when expressing fluid from an incision. In the illustrated embodiment, the beveled surface 50 is disposed on the expression cap 32, but it is contemplated that the beveled surface 50 can be located on other components. For instance, in another variation, the beveled surface 50 is located on the expression ring 38. In the illustrated embodiment, the expression ring 38 is in the form of a toroidal (doughnut shaped) ring, such as an o-ring, but it is envisioned that the expression ring 38 can be shaped differently in other embodiments.

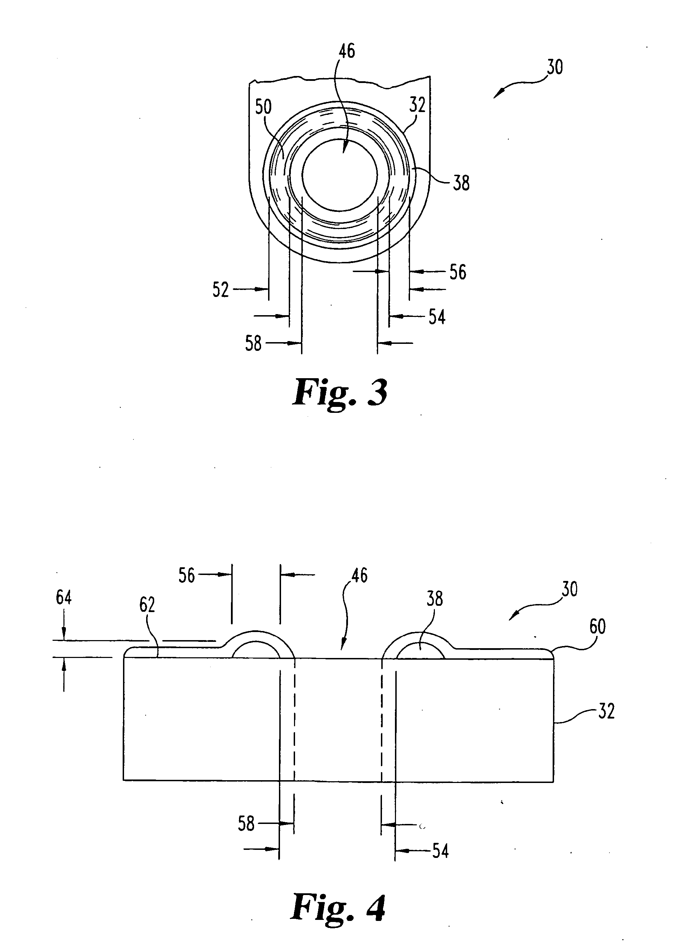

FIG. 3 provides a second top plan view of the expression device 30 in order to illustrate the various dimensions of the expression device 30. The dimensions illustrated in FIG. 3 include an outer diameter 52, an inner diameter 54, and a cross-sectional width 56 of the expression ring 38. In addition, FIG. 3 illustrates an outer diameter 58 of the expression opening 46. In one embodiment, the outer diameter 52 of the expression ring 38 is 10 mm, and the inner diameter 54 of expression ring 38 is 7 mm. Consequently, the cross-sectional width 56 of the expression ring 38 is 1.5 mm. In this embodiment, the internal diameter 58 of the expression opening 46 is 6 mm. It is envisioned that in other embodiments one or more of these dimensions can vary. Moreover, it should be recognized that, due to manufacturing tolerances, these dimensions can vary within a specified range.

FIG. 4 illustrates an overmold layer 60 of elastomeric material being deposited over the expression ring 38 as well as the face of the expression cap 32. The overmold layer 60 helps in expressing fluid from the incision. In one particular form, the overmold layer 60 is made from rubber, but it is envisioned that in other embodiments, the overmold layer 60 can be made from other materials. With continued reference to FIG. 4, the flange 40 of the expression cap 32 has a generally flat skin contacting surface or face 62 upon which the expression ring 38 is disposed. During expression of fluid from the incision, the skin contacting surface 62 faces the skin or other tissue in a generally parallel manner. Relative to the skin contacting surface 62, the expression ring 38 has a height 64 that the expression ring 38 extends from the skin contacting surface 62. In one embodiment, the height 64 of the expression ring 38 relative to the skin contacting surface 62 is approximately 0.5 mm. However, in other embodiments, this height 64 might vary.

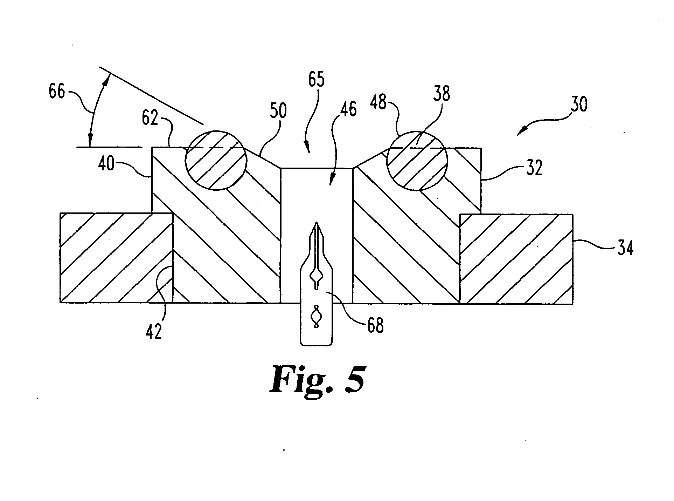

As can be seen in FIG. 5, the expression cap 32 has the beveled skin contacting surface 50 located between the expression ring 38 and the expression opening 46. The beveled surface 50 forms a concave depression 65 around the expression opening 46. Relative to the skin contacting surface 62, the beveled surface 50 has a countersink angle 66. In one form, the countersink angle 66 is between 20° to 30°. Unexpectedly, it was discovered that the countersink angle 66 of 20° provided exceptional results for expressing fluid.

**Table I has been provided below which shows the results of a control study.**

| Table I | | | | | | |
|---|---|---|---|---|---|---|
| Expression Ring | Lance Depth (mm) | Lancing Success Rate (%)* | Mean Blood Volume Expressed (µL) | Expression Success Rate (%)** | N | % Improvement (Expressed Blood) Over Control |
| Control | 1.8 | 100 | 0.233 | 88 | 15 | 0 |
| O-ring | 1.8 | 100 | 0.278 | 94 | 15 | 19.3 |
| Concave Ring (20°) | 1.8 | 100 | 0.578 | 100 | 15 | 148.1 |

| | | | | | | |
|---|---|---|---|---|---|---|
| * Lancing success is defined as ≥ 0.1 µL blood on the skin surface with or without (manual expression by finger squeeze) an expression ring ** Expression success rate is defined as ≥ 0.1 µL blood to the skin surface with the expression ring | | | | | | |

In the study protocol, three healthy subjects with normal skin types were lanced with a conventional microsampler 68 of a type commonly known in the art. The microsampler 68 used in the control study included a generally flat lancet with a capillary groove for transporting fluid to a test strip or sensor attached to the lancet. The lancet of the microsampler 68 lanced an incision in the skin with a single forward and backward movement through the expression or lancet opening 46. The microsampler 68 was fired using a conventional spring-based lancing mechanism, but it should be recognized that other types of lancing or firing mechanisms can be used to fire the lancet. Each subject was lanced to a depth of 1.8 mm on both hands as well as on the forearm and the radial side of the ring, middle, and index fingers.

Prior to lancing the skin, the subject pushed the incision site against one of three types of expression rings: a control type, an o-ring type, and a concave type. Fifteen (15) tests (N) were performed for each type of expression ring. The control type expression member was generally rigid and ring-shaped, and the surface that contacts the skin in the control was generally flat. With the o-ring type expression ring, the ring had an elastic o-ring that was pressed against the skin, but the o-ring type expression ring did not have a beveled surface around the expression opening. On the other hand, the concave type expression ring was configured in the manner as described above. That is, the concave expression ring included the expression cap 32 of the type illustrated in FIGS. 1 and 5. The expression cap 32 that was tested had a beveled surface 50 with a countersink angle 66 of 20°, and the expression cap 32 had the same dimensions as discussed above.

After lancing, the incision site was firmly held against each type of expression ring for 4 seconds. The finger was removed and blood on the surface of the skin was collected with a capillary tube. The volume of blood in the capillary tube was then measured. The results from the tests of the three expression ring types have been tabulated in Table I.

For the "Lancing Success Rate" column in Table I, lancing was considered successful when the sample volume of blood produced on the skin was at least 0.1 µL, regardless of the manner in which the sample volume was obtained. In other words, the "Lancing Success Rate" column in Table I reflects whether the incision was capable of ever producing a sufficient fluid sample. For example, if the microsampler did not cut enough capillaries beneath the skin to produce a sufficient sample size, then the lancing would have been considered a failure. Lancing success could have been achieved with or without the need for expressing fluid. So if the incision by itself bled a sufficient volume of blood, without any type of expression, the lancing would have been considered successful. Likewise, even if use of the expression ring was unable to produce a sufficient sample volume, but a sample volume ≥ 0.1 µL was afterwards created by expressing in some other manner, such as by pinching or squeezing the skin, then lancing was considered successful. The 0.1 µL cutoff value was selected because 0.1 µL sample volume sizes are typically the lower limit for accurate home diagnostic testing in the current state of the art. The "Mean Blood Volume Expressed" column in Table I indicates the mean blood volume collected over all of the test for a particular expression ring type. Regarding the "Expression Success Rate" column in Table I, expression with a selected expression ring type was considered successful when the sample volume of blood on the skin surface was ≥ 0.1 µL. It should be recognized that the differences between the "Lancing Success Rate" and "Expression Success Rate" columns occurred when the particular expression ring could not express a sufficient amount of blood, but a sufficient amount (i.e., ≥ 0.1 µL) was able to be expressed in some other manner, such as by squeezing the skin. As can be seen in Table I, the expression cap 32 with countersink angle 66 of 20° (concave ring type) was able to successfully express fluid every time, whereas the control and o-ring expression ring types were considerably less successful.

Unexpectedly, it was discovered that the expression cap 32 with countersink angle 66 of 20° was able to produce over twice as much blood as compared to the control and o-ring expression ring types. The "Percent (%) Improvement (Expressed Blood) Over Control" column shows the percentage difference in volume between the expression ring being tested and the control. Looking at Table I, with the expression cap 32 having a countersink angle 66 of 20°, the percent improvement over the control expression ring was significant, specifically 148.1 %. Even when this concave ring type is compared with the o-ring type expression ring, the concave ring type with a 20° countersink angle 66 represented about a 107.9 % improvement in mean blood volume expressed. Thus, it should be appreciated that the concave expression ring enhances the ability to consistently provide a sufficient fluid sample for testing purposes.

## Claims

1. An expression device (30), comprising:
an expression cap (32) defining a lancet opening (46);
the expression cap (32) having a skin contacting surface that is generally flat and positioned to face tissue during expression of body fluid;
the expression cap (32) including an elastomeric ring (38) extending around the lancet opening (46) from the skin contacting surface, wherein the elastomeric ring (38) includes elastomeric material; and
the expression cap (32) having a beveled surface (50) between the lancet opening and the elastomeric ring (38) to enhance expression of the body fluid when the expression cap (32) is pressed around an incision site, and an overmold layer (60) covering the elastomeric ring (38), **characterized in that** inside the elastomeric ring (38) the expression cap (32) has a beveled surface (50).

2. The device of claim 1, wherein the beveled surface extends at a 20° to 30° angle relative to the skin contacting surface.

3. The device of claim 1, wherein the beveled surface extends at a 20° angle relative to the skin contacting surface.

4. The device of claim 2, wherein:
the elastomeric ring has a outer diameter of 10 mm;
the elastomeric ring has an inner diameter of 7 mm;
the elastomeric ring has a height of 1.5 mm relative to the skin contacting surface; and
the lancet opening has an internal diameter of 6 mm.

5. The device of claim 1, wherein the elastomeric ring is an o-ring.

6. The device of claim 1, wherein the expression cap is generally ring-shaped.

## Patentansprüche

1. Expressionsvorrichtung (30), die Folgendes umfasst:
eine Expressionskappe (32), die eine Lanzettenöffnung (46) definiert;
wobei die Expressionskappe (32) eine Hautkontaktfläche aufweist, die im Allgemeinen flach ist und so angeordnet ist, dass sie beim Ausdrücken von Körperflüssigkeit zum Gewebe zeigt;
wobei die Expressionskappe (32) einen elastomeren Ring (38) aufweist, der sich von der Hautkontaktfläche um die Lanzettenöffnung (46) herum erstreckt, worin der elastomere Ring (38) ein elastomeres Material aufweist; und worin die Expressionskappe (32) eine abgeschrägte Fläche (50) zwischen der Lanzettenöffnung und dem elastomeren Ring (38) aufweist, um die Expression der Körperflüssigkeit zu verbessern, wenn die Expressionskappe (32) um die Einschnittstelle gedrückt wird, und worin eine Umspritzungsschicht (60) den elastomeren Ring (38) bedeckt, **dadurch gekennzeichnet, dass** die Expressionskappe (32) im elastomeren Ring (38) eine abgeschrägte Fläche (50) aufweist.

2. Vorrichtung nach Anspruch 1, worin sich die abgeschrägte Fläche in einem Winkel von 20° bis 30° relativ zur Hautkontaktfläche erstreckt.

3. Vorrichtung nach Anspruch 1, worin sich die abgeschrägte Fläche in einem Winkel von 20° relativ zur Hautkontaktfläche erstreckt.

4. Vorrichtung nach Anspruch 2, worin:
der elastomere Ring einen Außendurchmesser von 10 mm aufweist;
der elastomere Ring einen Innendurchmesser von 7 mm aufweist;
der elastomere Ring eine Höhe von 1,5 mm relativ zur Hautkontaktfläche aufweist; und
die Lanzettenöffnung einen Innendurchmesser von 6 mm aufweist.

5. Vorrichtung nach Anspruch 1, worin der elastomere Ring ein O-Ring ist.

6. Vorrichtung nach Anspruch 1, worin die Expressionskappe im Allgemeinen ringförmig ist.

## Revendications

1. Dispositif d'expression (30), comprenant :
une coiffe d'expression (32) définissant une ouverture de lancette (46) ;
la coiffe d'expression (32) ayant une surface de contact avec la peau qui est de manière générale plate et positionnée de manière à faire face au tissu lors de l'expression d'un fluide corporel ;
la coiffe d'expression (32) incluant une bague élastomérique (38) s'étendant autour de l'ouverture de lancette (46) à partir de la surface de contact avec la peau, dans lequel la bague élastomérique (38) inclut un matériau élastomérique ; et
la coiffe d'expression (32) ayant une surface biseautée (80) entre l'ouverture de lancette et la bague élastomérique (38) pour améliorer l'expression du fluide corporel lorsque la coiffe d'expression (32) est pressée autour d'un site d'incision, et une couche surmoulée (60) recouvrant la bague élastomérique (38), **caractérisé en ce qu'**à l'intérieur de la bague élastomérique (38), la coiffe d'expression (32) a une surface biseautée (50).

2. Dispositif selon la revendication 1, dans lequel la surface biseautée s'étend selon un angle de 20° à 30° par rapport à la surface de contact avec la peau.

3. Dispositif selon la revendication 1, dans lequel la surface biseautée s'étend selon un angle de 20° par rapport à la surface de contact avec la peau.

4. Dispositif selon la revendication 2, dans lequel :
la bague élastomérique a un diamètre extérieur de 10 mm ;
la bague élastomérique a un diamètre intérieur de 7 mm ;
la bague élastomérique a une hauteur de 1,5 mm par rapport à la surface de contact avec la peau ; et
l'ouverture de lancette a un diamètre interne de 6 mm.

5. Dispositif selon la revendication 1, dans lequel la bague élastomérique est une bague toroïdale.

6. Dispositif selon la revendication 1, dans lequel la coiffe d'expression est de façon générale en forme de bague.
